# EUROPEAN PATENT APPLICATION

(11) **EP 4 140 471 A1**
(43) Date of publication of application: **01.03.2023**
(21) Application number: 21194184.4
(22) Date of filing: 31.08.2021
(51) Int. Cl.: A61K 8/58, A61K 8/60, A61K 8/64, A61K 8/86, A61Q 19/08

(54) **COMPOSITION WITH BIOLOGICALLY ACTIVE AGENT**

(71) Applicant: The Boots Company plc, Nottingham NG90 1BS (GB)
(72) Inventor: JOHNSON, Mark, Nottingham, NG15 8DQ (GB); ELMS, Beverley Jane, Nottingham, NG13 0FH (GB); TOMLINSON, Paul James, Nottingham, DE22 1EX (GB)
(74) Representative: Teasdale, Andrew James

(57) **Abstract**

Compositions, and methods and uses thereof are provided, wherein the compositions comprise a biologically active agent and one or more solvents that dissolve the biologically active agent.

## Description

### Technical Field

The present invention relates to a composition comprising an active agent and a solvent that is designed to improve delivery of the active agent into the epidermis. The invention also extends to a method of delivering a composition of the invention to the skin of a subject, and the use of one or more solvents to dissolve a biologically active peptide.

### Background to the invention

Biologically active agents, such as peptides have been used in cosmetic compositions and products for several years to stimulate peptide upregulation, skin repair and regeneration. The most commonly used peptide is the combination of palmitoyl oligopeptide (Pal-GHK), which acts as a messenger peptide for collagen renewal and consists of a sequence derived from collagen I, and palmitoyl tetrapeptide-7 (Pal-GQPR), which reduces the production of the inflammatory cytokine, interleukin-6 (IL-6), and inhibits extracellular matrix (ECM) degradation. This said peptide combination is available from Sederma under the trade name Matrixyl.

However, some biologically active agents, such as peptides, have limited solubility in the skin due to their hydrophilic nature. In order to address this problem, peptides have been modified to make them more lipophilic. For example, by covalently attaching a lipophilic group, such as a fatty acid (e.g., palmitic acid), to the peptide. Such modified peptides are better absorbed by the skin (compared to the non-modified peptides) because the skin is generally lipophilic and/or hydrophobic in nature.

Whilst natural and synthetic agents have been in use for a number of years there remains a need to identify new and improved ways of delivering biologically active agents into the skin.

### Summary of the invention

Thus, according to the present invention, there is provided a composition comprising a biologically active agent and one or more solvents, which dissolve the biologically active agent, selected from the group comprising a dimethyl silane, a monosaccharide ether, and mixtures thereof.

Advantageously, the solvent of the invention ensures that the biologically active agent is stable within the composition while also encouraging a significant improvement in the delivery of the biologically active agent into skin.

According to another aspect of the invention, there is provided a method of delivering or administering a composition of the invention to the skin of a subject. The method may comprise contacting a composition according to the invention with the skin of said subject.

According to another aspect of the invention, there is provided a use of one or more solvents of the invention to dissolve a biologically active agent.

### Detailed description of the invention

The present invention is drawn to a composition comprising a biologically active agent (e.g., a peptide) and at least one solvent that improves the solubility of the biologically active agent within the composition, and thus also improves the delivery of the biologically active agent to the epidermis.

### Solvent

A composition of the invention may be applied to surface of the skin in order for the composition, particularly the biologically active agent (e.g., peptide), to be absorbed by the epidermis. However, some agents (e.g., peptides) are extremely hydrophilic and thus not readily absorbed by the skin, as the skin is generally hydrophobic in nature.

The solvent referred to herein increases the solubility of the biologically active (e.g., peptide) in the composition, and thus improves the penetration of the composition, particularly the active agent, into the epidermis. This improvement in the transfer of the biologically active agent into the epidermis provides improved efficiency in the delivery of the active agent to the epidermis, particularly, the stratum corneum (SC) of the epidermis.

The solvent of the present invention also improves penetration into the stratum corneum and partitions the biologically active agent(s) into the most efficient location across the epidermis. The solvent does not encourage the composition, particularly the biologically active agent, to pass through the epidermis into the dermis. The solvent does not encourage the composition, particularly the active agent, to remain on the surface of the epidermis.

The solvent according to the invention may be a dimethyl silane. The solvent according to the invention may be a monosaccharide ether.

The dimethyl silane referred to herein is a water-soluble silicone. It is also clear in water (i.e., without the appearance of oil droplets or a hazy appearance). The dimethyl silane may be or comprise a silicone wax or a silicone oil. A silicone wax refers to a silicone wax with a melting point above 39°C. A silicone oil refers to a siloxane polymer that is a liquid at 25°C and at atmospheric pressure (760 mmHg). The dimethyl silane may be or comprise an ether of dimethyl silane (ether dimethyl silane). Preferably the ether of the dimethyl silane is or comprises a PEG methyl ether dimethyl silane, such as a bis-PEG methyl ether dimethyl silane. Most preferably the dimethyl silane is or comprises bis-PEG-18 methyl ether dimethyl silane. The formula of bis-PEG-18 methyl ether dimethyl silane is provided herein as Formula (I) as follows:

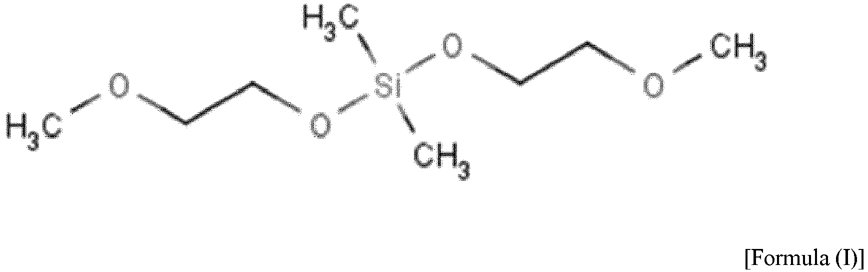

The monosaccharide ether referred to herein may be used as a humectant. The monosaccharide ether may be or comprise a triose ether, a pentose ether, a hexose ether or a heptose ether. Preferably the monosaccharide ether is or comprises a hexose ether, such as a glucose ether. The monosaccharide ether may be or comprise an alkoxylated alkyl monosaccharide ether. The alkoxylate may be C1 to C5. The alkyl may be C1 to C5. Preferably, the alkoxylate is C2 and the alkyl is C1. Preferably, therefore, the alkoxylated alkyl monosaccharide ether is an ethoxylated methyl monosaccharide ether. The alkoxylated monosaccharide ether (e.g., an ethoxylated methyl monosaccharide ether) may be or comprise an alkoxylated alkyl triose ether, an alkoxylated alkyl pentose ether, an alkoxylated alkyl hexose ether or an alkoxylated alkyl heptose ether. Preferably the alkoxylated alkyl monosaccharide ether is or comprises an alkoxylated alkyl hexose ether. More preferably the alkoxylated hexose ether is or comprises an alkoxylated alkyl glucose ether, such as an ethoxylated methyl glucose ether. Preferably, the ethoxylated methyl glucose ether is or comprises methyl-gluceth-20. Methyl-gluceth-20 is an extremely effective humectant. The formula of methyl-gluceth-20 is provided herein as Formula (II) as follows:

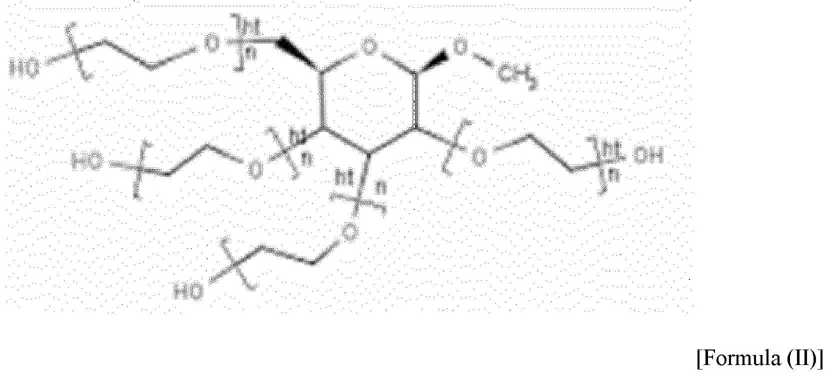

Thus, in one embodiment, the solvent according to the invention may be one or more solvents selected from the group consisting of: an ether of dimethyl silane; a monosaccharide ether; and mixtures thereof.

The composition according to the invention may further comprise one or more members selected from the group consisting of: solvents (such as glycerides, sorbitan esters, polyglycerin esters, non-silicone fatty compounds, and esters), water-soluble solvents, surfactants (such as sorbitan esters and polyglycerin esters) and mixtures thereof.

Thus, the composition according to the invention may further comprise one or more solvents selected from the group consisting of: glycerides, sorbitan esters, polyglycerin esters, non-silicone fatty compounds, esters, and mixtures thereof.

The term *"water-soluble solvent"* is interchangeable with the term *"water-miscible solvent"* and means a compound that is liquid at 25°C and at atmospheric pressure (760 mmHg), and it has a solubility of at least 50% in water under these conditions. In some cases, the water-soluble solvent has a solubility of at least 60%, 70%, 80%, or 90% in water. The solvents can be volatile or non-volatile compounds.

Non-limiting examples of water-soluble solvents may be one or more members selected from the group consisting of glycerin, alcohols (for example, C₁₋₃₀, C₁₋₁₅, C₁₋₁₀, or C₁₋₄ alcohols), organic solvents, polyols (polyhydric alcohols), glycols (e.g., butylene glycol, caprylyl glycol, etc.), and mixtures thereof.

Also suitable are one or more glycols and polyols selected from the group consisting of monomethyl, monoethyl and monobutyl ethers of ethylene glycol, propylene glycol, hexylene gycol or ethers thereof such as, for example, monomethyl ether of propylene glycol, butylene glycol, hexylene glycol, dipropylene glycol as well as alkyl ethers of diethylene glycol, , 1,2,6-hexanetriol, trimethylolpropane, diethylene glycol, triethylene glycol, tetraethylene glycol, pentaethylene glycol, 2-butene-1,4-diol, 2-ethyl-1,3-hexanediol, 2-methyl-2,4-pentanediol, (caprylyl glycol), 1,2-hexanediol, 1,2-pentanediol, and 4-methyl-1,2-pentanediol; ethylene glycol monomethyl ether, ethylene glycol monoethyl ether, ethylene glycol monobutyl ether, ethylene glycol monomethyl ether acetate, diethylene glycol monomethyl ether, diethylene glycol monoethyl ether, diethylene glycol mono-n-propyl ether, ethylene glycol mono-iso-propyl ether, diethylene glycol mono-iso-propyl ether, ethylene glycol mono-n-butyl ether, ethylene glycol mono-t-butyl ether, diethylene glycol mono-t-butyl ether, 1-methyl-1-methoxybutanol, propylene glycol monomethyl ether, propylene glycol monoethyl ether, propylene glycol mono-t-butyl ether, propylene glycol mono-n-propyl ether, propylene glycol mono-iso-propyl ether, dipropylene glycol monomethyl ether, dipropylene glycol monoethyl ether, dipropylene glycol mono-n-propyl ether, and dipropylene glycol mono-iso-propyl ether; 2-pyrrolidone, N-methyl-2-pyrrolidone, 1,3-dimethyl-2-imidazolidinone, formamide, acetamide, dimethyl sulfoxide, sorbit, sorbitan, acetine, diacetine, triacetine, sulfolane, and mixtures thereof.

Also suitable are one or more polyhydric alcohols selected from the group consisting of tripropylene glycol, 1,3-butanediol, 2,3-butanediol, 1,4-butanediol, 3-methyl-1,3-butanediol, 1,5-pentanediol, tetraethylene glycol, 1,6-hexanediol, 2-methyl-2,4-pentanediol, polyethylene glycol, 1,2,4-butanetriol, 1,2,6-hexanetriol, and mixtures thereof. Polyol compounds may also be used e.g., ethoxylation of polyols producing methyl gluceth-20. Ethoxylated water dispersible waxes may be used e.g., Bis PEG-18 methyl ether dimethyl silane.

The composition may further comprise a surfactant. If the composition comprises a surfactant then the surfactant will be present in excess of any surfactant necessary to create an emulsion composition. Suitable surfactants may be selected from the group consisting of anionic, non-ionic, cationic and/or amphoteric or zwitterionic surfactants.

Typical examples of anionic surfactants are soaps, alkylbenzene sulphonates, alkane sulphonates, olefin sulphonates, alkylether sulphonates, glycerin ether sulphonates, α-methylester sulphonates, sulphonated fatty acids, alkyl sulphates, alkylether sulphates, glycerin ether sulphates, fatty acid ether sulphates, mixed ether/hydroxyl sulphates, monoglycerin ether sulphates, fatty acid amide ether sulphates, mono- and dialkylsulphonates, mono- and dialkylsulphosuccinamates, sulphoglycerides, amide soaps, ether carbonic acids and the salts thereof, fatty acid isethionates, fatty acid sarcocinates, fatty acid taurides, N-acyl amino acids, such as, for example, acyl lactilate, acyl tartrate, acyl glutamate and acyl asparate, alkyloligoglucoside sulphate, protein-carrying fatty acid condensates (in particular, vegetable wheat-based products) and alkyl(ether) phosphates.

Typical examples of non-ionic surfactants are fatty alcohol polyglycol ether, alkyl phenol polyglycol ether, fatty acid polyglycol ester, fatty acid amide polyglycol ether, fatty amino polyglycol ether, alkoxylated triglycerides, mixed ethers or mixed formals, optionally partially oxidised alkylenyl oligoglycosides or derivatives of glucoronic acid, fatty acid N-alkyl glucamides, protein hydrolysates (in particular, vegetable wheat-based products), polyol fatty acid esters, sugar esters, sorbitan esters, polysorbate esters and amino oxide esters e.g., PEG-20 Methyl Glucose Sesquistearate and polysorbate-20.

Further suitable surfactants include addition products of from 15 to 60 mol of ethylene oxide to castor oil and/or hydrogenated castor oil; partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide; partial esters of polyglycerol (average degree of self-condensation 2 to 8), polyethylene glycol (molecular weight 400 to 5 000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide; mixed esters of pentaerythritol, fatty acids, citric acid and fatty alcohol and/or mixed esters of fatty acids having 6 to 22 carbon atoms, methylglucose and polyols, preferably glycerol or polyglycerol.

The composition may further comprise ethylene oxide and/or of propylene oxide to fatty alcohols, fatty acids, alkylphenols or to castor oil are known, commercially available products. These are homologue mixtures whose average degree of alkoxylation corresponds to the ratio of the amounts of substance of ethylene oxide and/or propylene oxide and substrate with which the addition reaction is carried out. C12/18-fatty acid mono- and diesters of addition products of ethylene oxide to glycerol are known as refatting agents for cosmetic preparations.

The solvent may further comprise a volatile or non-volatile sorbitan esters including sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitane trihydroxystearate, sorbitan monotartrate, sorbitan sesquitartrate, sorbitan ditartrate, sorbitan tritartrate, sorbitan monocitrate, sorbitan sesquicitrate, sorbitan dicitrate sorbitan tricitrate, sorbitan monomaleate, sorbitan sesquimaleate, sorbitan dimaleate, sorbitan trimaleate as well as the technical admixtures thereof.

Additional suitable solvents may be polyglycerin esters including polyglyceryl-2 dipolyhydroxystearate (Dehymuls^{®} PGPH), polyglycerin-3-diisostearate (Lameform^{®} TGI), polyglyceryl-4 isostearate (Isolan^{®} GI 34), polyglyceryl-3 oleate, diisostearoyl polyglyceryl-3 diisostearate (Isolan^{®} PDI), polyglyceryl-3 methylglucose distearate (Tego Caret 450), polyglyceryl-3 beeswax (Cera Bellina^{®}), polyglyceryl-4 caprate (Polyglycerol Caprate T2010/90), polyglyceryl-3 cethyl ether (Chimexane^{®} NL), polyglyceryl-3 distearate (Cremophor^{®} GS 32) and polyglyceryl polyricinoleate (Admul^{®} WOL 1403), polyglyceryl dimerate isostearate, as well as admixtures thereof. Examples of polyol ester which are also suitable are the mono-, di- and triesters of trimethylolpropane or pentaerythritol which may be optionally reacted with 1 to 30 moles of ethylene oxide, of trimethylolpropane or pentaerythritol with lauric acid, fatty coconut acid, fatty tallow acid, palm acid, stearic acid, oleic acid, behenic acid and the like.

A further solvent may be an emollient, such as one or more members selected from the group consisting of: PEG/PPG-8/Laurate, Polygyceryl-3 laurate,PEG-7 Glyceryl Cocoate, PPG-15 stearyl ether, ethylhexyl stearate, cetyl dimethicone, octyldodecanol, PPG-20 methyl glucose ether, isopropyl myristate isopropyl palmitate, isopropyl laurate isodecyl laurate, isodecyl neopentanoate, isohexadecane, pentaerythrityl tetraisostearate, caprylic/capric triglyceride, canola oil, sunflower oil (Helianthus annuus), olive oil (Olea europea), cottonseed oil (Gossypium herbaceum), jojoba oil (Simmondsia chinensis), shea butter (Butyrospermum parkii), cocoa butter (Theobroma cacao), cupuacu butter (Theobroma grandiflorum), avocado oil (Persea gratissima), liquid paraffin, dimethicone, phenyl trimethicone, cyclopentasiloxane, dimethiconol, petrolatum, C12-15 alcohol benzoate, Propylene Glycol Ricinolate, glyceryl isostearate, and mixtures thereof.

Preferably, the solvent is selected from the group consisting of: methyl gluceth-20, bis-PEG-18 methyl ether dimethyl silane, and mixtures thereof.

In some embodiments, the solvent may be present in the composition at a concentration ranging from 0.5% to 95%, from 1% to 90%, from 1.5% to 70%, from 2% to 50%, from 2% to 40%, from 2% to 30%, from 2% to 25%, from 0.5 % to 5 %, from 1 % to 3%, from 1% to 2%, from 40% to 70%, from 45% to 60%, or from 48% to 52%, based on total weight of the composition.

### Biologically active agent

The biologically active agent may be a vitamin, an antibiotic, a steroid, an immunosuppressant (e.g., a macrolide), a glycosaminoglycan (e.g., hyaluromic acid), a phenol (e.g., salicylic acid or hydroquinone), a peroxide, urea, lactic acid, or a peptide.

Preferably, the biologically active agent is a peptide. The biologically active peptide may be a dipeptide, a tripeptide, a tetrapeptide, or a pentapeptide. Preferably, the biologically active peptide is a tetrapeptide.

The peptide, such as a tetrapeptide, may be a matrikine. It will be appreciated that a matrikine is a signalling peptide that upregulates the production of proteins of the extracellular matrix (ECM), including at least fibrillin, fibronectin, decorin and collagen IV. Matrikines can be produced naturally within skin by the fragmentation of ECM proteins or synthesised.

The peptide, such as a tetrapeptide or a matrikine, may or may not be a lipo-peptide. A lipo-peptide is a peptide connected (covalently) to a lipid. The lipid component of the lipo-peptide may be a fatty acid selected from the group consisting of: palmitic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S. Preferably, the lipid component of the lipo-peptide is palmitic acid. Preferably, the biologically active peptide is a lipo-matrikine (e.g., a palmityl matrikine). The lipo-matrikine may be a tetrapeptide.

The peptide (e.g. a lipo-peptide) may have an amino acid sequence selected from the group consisting of: (i) GPXG (SEQ ID No. 1), (ii) LSXX (SEQ ID No. 2), (iii) XXGD (SEQ ID No. 3), or (iv) QTAV (SEQ ID No. 4),
wherein for (i) G denotes the amino acid glycine and P denotes the amino acid proline (as per the internationally recognised single letter code for amino acids), and X denotes an amino acid selected from the group consisting of Lysine (K), Glutamic acid (E) and Serine (S) and mixtures thereof,
wherein for (ii) L denotes the amino acid Leucine and S denotes the amino acid Serine (as per the internationally recognised single letter code for amino acids), and X denotes an amino acid selected from the group consisting of Valine (V), Aspartic acid (D), Proline (P), Glycine (G) and mixtures thereof,
wherein for (iii) G denotes the amino acid Glycine and D denotes the amino acid Aspartic acid (as per the internationally recognised single letter code for amino acids), and X denotes an amino acid selected from the group consisting of Glutamic acid (E), Lysine (K), Leucine (L), Alanine (A), Isoleucine (I), Arginine (R) and mixtures thereof, and
wherein for (iv) Q denotes the amino acid Glutamine, T denotes the amino acid Threonine, A denotes the amino acid Alanine, and V denotes the amino acid Valine.

In another embodiment, the peptide (e.g. a lipo-peptide) has an amino acid sequence selected from the group consisting of: (i)Y-GPXG-Z (SEQ ID No. 5), (ii) Y-LSXX-Z (SEQ ID No. 6), (iii) Y-XXGD-Z (SEQ ID No. 7), or (iv) Y-QTAV-Z (SEQ ID No. 8),
wherein for (i) G denotes the amino acid glycine and P denotes the amino acid proline (as per the internationally recognised single letter code for amino acids), and X denotes an amino acid selected from the group consisting of Lysine (K), Glutamic acid (E) and Serine (S) and mixtures thereof,
wherein for (ii) L denotes the amino acid Leucine and S denotes the amino acid Serine (as per the internationally recognised single letter code for amino acids), and X denotes an amino acid selected from the group consisting of Valine (V), Aspartic acid (D), Proline (P), Glycine (G) and mixtures thereof,
wherein for (iii) G denotes the amino acid Glycine and D denotes the amino acid Aspartic acid (as per the internationally recognised single letter code for amino acids), and X denotes an amino acid selected from the group consisting of Glutamic acid (E), Lysine (K), Leucine (L), Alanine (A), Isoleucine (I), Arginine (R) and mixtures thereof,
wherein for (iv) Q denotes the amino acid Glutamine, T denotes the amino acid Threonine, A denotes the amino acid Alanine, and V denotes the amino acid Valine, and
wherein at the N-terminal end, Y is selected from the group consisting of H, -CO-R¹, -SO₂-R¹ or a biotinyl group, at the C-terminal end, Z is selected from the group consisting of OH, O R¹, NHR¹ or NR¹R².

In one embodiment, R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a preferred embodiment of the present invention the tetrapeptide is modified at the N-terminal and/or the C-terminal end.

In a preferred embodiment the tetrapeptides of the present invention is selected from the group consisting of: Y-EKGD-Z (SEQ ID No. 9), Y-ELGD-Z (SEQ ID No. 10), Y-EAGD-Z (SEQ ID No. 11), Y-EIGD-Z (SEQ ID No. 12), Y-ERGD-Z (SEQ ID No. 13), Y-KEGD-Z (SEQ ID No. 14), Y-KLGD-Z (SEQ ID No. 15), Y-KAGD-Z (SEQ ID No. 16), Y-KIGD-Z (SEQ ID No. 17), Y-KRGD-Z (SEQ ID No. 18), Y-LEGD-Z (SEQ ID No. 19), Y-LKGD-Z (SEQ ID No. 20), Y-LAGD-Z (SEQ ID No. 21), Y-LIGD-Z (SEQ ID No. 22), Y-LRGD-Z (SEQ ID No. 23), Y-IEGD-Z (SEQ ID No. 24), Y-IKGD-Z (SEQ ID No. 25), Y-ILGD-Z (SEQ ID No. 26), Y-IAGD-Z (SEQ ID No. 27), Y-IRGD-Z (SEQ ID No. 28), Y-REGD-Z (SEQ ID No. 29), Y-RKGD-Z (SEQ ID No. 30), Y-RLGD-Z (SEQ ID No. 31), Y-RAGD-Z (SEQ ID No. 32), Y-RIGD-Z (SEQ ID No. 33), Y-AEGD-Z (SEQ ID No. 34), Y-AKGD-Z (SEQ ID No. 35), Y-ALGD-Z (SEQ ID No. 36), Y-AIGD-Z (SEQ ID No. 37) and Y-ARGD-Z (SEQ ID No. 38). In another embodiment, the tetrapeptides is selected from the group consisting of Y-EKGD-Z (SEQ ID No. 9), Y-LKGD-Z (SEQ ID No. 20), Y-IRGD-Z (SEQ ID No. 28) and Y-AKGD-Z (SEQ ID No. 35). In another embodiment the tetrapeptide is Y-EKGD-Z (SEQ ID No. 9). In another embodiment the tetrapeptide is Y-LKGD-Z (SEQ ID No. 20). In another embodiment the tetrapeptide is Y-IRGD-Z (SEQ ID No. 28). In another embodiment the tetrapeptide is Y-AKGD-Z (SEQ ID No. 35).

In a preferred embodiment the tetrapeptide combination of the present invention, tetrapeptide a) is selected from the group consisting of: Y-LSVD-Z (SEQ ID No. 39), Y-LSVP-Z (SEQ ID No. 40), Y-LSVG-Z (SEQ ID No. 41), Y-LSDV-Z (SEQ ID No. 42), Y-LSDP-Z (SEQ ID No. 43), Y-LSDG-Z (SEQ ID No. 44), Y-LSPV-Z (SEQ ID No. 45), Y-LSPD-Z (SEQ ID No. 46), Y-LSPG-Z (SEQ ID No. 47), Y-LSGV-Z (SEQ ID No. 48), Y-LSGD-Z (SEQ ID No. 49) and Y-LSGP-Z (SEQ ID No. 50). In another embodiment, the tetrapeptides is selected from the group consisting of Y-LSVD-Z (SEQ ID No. 39), Y-LSPG-Z (SEQ ID No. 47) and Y-LSPD-Z (SEQ ID No. 46). In another embodiment the tetrapeptide is Pal-LSVD-OH (SEQ ID No. 51). In another embodiment the tetrapeptide is Pal-LSPG-OH (SEQ ID No. 52). In another embodiment the tetrapeptide is Pal-LSPD-OH (SEQ ID No. 53).

In a further preferred embodiment of the present invention, the tetrapeptide is Y-GPKG-Z (SEQ ID No. 54). In a further preferred embodiment of the present invention the tetrapeptide is Y-GPEG-Z (SEQ ID No. 55). In a further preferred embodiment of the present invention the tetrapeptide is Y-GPSG-Z (SEQ ID No. 56).

The matrikine may be or comprise GHK (e.g., Pal-GHK) and/or GQPR (e.g., Pal-GQPR).

Where, at the N-terminal, Y is H then the amino acid is not modified. When, at the C-terminal, Z is OH then the amino acid is not modified. The tetrapeptide is thus not in derivatised form. When other than Y is H and Z is OH, then the tetrapeptide is derivatised. Derivation of the tetrapeptide is intended to increase the bioavailability of the peptide by improving the ability of the tetrapeptide to pass through the skin. An increase in bioavailability can also be achieved through encapsulation, micro-emulsions or through vectoring that increase the peptide delivery to the skin.

In a preferred embodiment of the present invention, R¹ and/or R² is an alkyl chain of from 1 to 24 carbon atoms, preferably a lipophilic alkyl chain of 3 to 24 carbon atoms.

In a further preferred embodiment of the present invention Y is an acyl group -CO-R¹ and Z is selected from the group consisting of OH, methoxy, ethoxy and NH₂. Z is preferably OH. In a further embodiment, Y is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment of the present Y is selected from lauroyl (C12), myristoyl (C14) and palmitoyl (C16).

In a further preferred embodiment Z is OH and Y is independently selected from the group consisting of palmitoyl (C16), myristoyl (C14) and lauroyl (C12). Most preferably Y is palmitoyl (C16) and Z is OH.

The tetrapeptides may comprise amino acids in the D- or L- configuration. The tetrapeptides may comprise an acid C-terminus such as -CO2H. Alternatively, the tetrapeptide may comprise an amide C-terminus such as -CONH2, -CONHR or CONR2, wherein R is an alkyl chain of preferably from 1 to 24 carbon atoms.

The amino acids making up the tetrapeptides according to the invention may be optically pure, be made up of L or D isomers or a mixture of them. L isomers are those present in the natural state and may be preferred.

The present invention also envisages further derivatives of the tetrapeptide, including for example modification and/or addition of a chemically functional group to one or more of the amino acids but without a change in the carbon skeletal. The present invention also envisages further analogues of the tetrapeptide, including modification and/or addition of a chemically functional group to one or more of the amino acids with a change in the carbon skeletal and complexes of the tetrapeptide with other species such as a metal ion (e.g., copper, zinc, manganese, magnesium, and others).

Tetrapeptides may be found in the form of salts, including hydrochloric salt, or acetate.

In one embodiment, the invention is a composition comprising a biologically active peptide (e.g., a lipo-matrikine or a tetrapeptide) and one or more solvents selected from the group consisting of: a dimethyl silane (e.g., an ether of dimethyl silane), a monosaccharide ether (e.g., methyl gluceth-20), and mixtures thereof.

In one embodiment, the invention is a composition comprising a lipo-matrikine and one or more solvents selected from the group consisting of a dimethyl silane, an ethyoxylated monosaccharide ether, and mixtures thereof.

Thus, in one embodiment, the invention is a composition comprising a lipo-matrikine and one or more solvents selected from the group comprising bis PEG-18 methyl ether dimethyl silane; methyl gluceth-20; and mixtures thereof.

In a preferred embodiment of the present invention the tetrapeptide is any of the tetrapeptide referred to herein that has not been modified at the N-terminal and/or the C-terminal end. Thus, the peptide does not comprise a Y at the N-terminal end and/or a Z at the C-terminal end.

### Excipient composition

The biologically active peptides of the present invention are provided by the supplier to the composition formulator as a concentrated excipient composition. The excipient composition is therefore a composition ingredient. The excipient composition comprises biologically active peptides at from 250 to 1650ppm. In an alternative embodiment, the excipient composition comprises from 0.1 to 50,000ppm. In a further alternative embodiment, the excipient composition comprises from 1 to 5,000ppm. In a further alternative embodiment, the excipient composition comprises from 10 to 500ppm.

The excipient composition, in addition to biologically active peptides, may optionally comprise one or more ingredients selected from the group consisting of: water, surfactants, diols, triols, glycerine, thickener and mixtures thereof. All suitable surfactants, diols (also known as glycols), triols, glycerine and thickener ingredients may be incorporated into the present excipient composition. Preferred surfactants for the excipient composition are selected from the group consisting of alkyl polyglucosides (preferably decyl glucoside, coco glucoside, lauryl glucoside), sodium lauroyl lactylate, polysorbate 20, polysorbate 60, sorbitan laurate, PEG/PPG-18/18 dimethicone, Cetyl PEG/PPG-10/1 dimethicone, Polyglyceryl-4 isostearate, Hexyl laurate, steareth-21, steareth-2, and mixtures thereof.

Preferred diols are selected from the group consisting of; pentylene glycol, caprylyl glycol, butylene glycol, di-propylene glycol, ethylhexylglycerine, propanediol, hexenediol, glycerol, butylene glycol, propylene glycol, isoprene glycol, dipropylene glycol, pentylene glycol, hexylene glycol, polypropylene glycol, butylene glycol, polyethylene glycol, sorbitol, glucitol, mannitol, hydroxypropyl sorbitol, erythritol, threitol, pentaerythritol, xylitol, and mixtures thereof.

Preferred triols are selected from the group consisting of: hexanetriol, glycerine, ethoxylated glycerin, propoxylated glycerin, and mixtures thereof.

Preferred thickeners are selected from the group consisting of: xanthan gum, ammonium acryloyldimethyltaurate/vinyl pyrrolidone copolymer, dimethicone crosspolymer, carbomer, hydroxyethyl cellulose, polyacrylamide, sodium polyacrylate, polyacrylate crosspolymer-6, acrylates/beheneth-25 methacrylate copolymer, Acrylates/C10-30 alkyl acrylate crosspolymer, and mixtures thereof.

### Cosmetic Composition

The composition of the invention is preferably a cosmetic composition, such as a skin care composition, e.g., a facial skincare cosmetic composition. Thus, the present invention also encompasses cosmetic compositions comprising one or more solvents of the present invention (e.g., a dimethyl silane and/or a monosaccharide ether) and a biologically active peptide.

The cosmetic composition of the present invention may be aqueous or non-aqueous and comprise a single-phase system or multiple phase system. The composition may include but is not limited to liquids, gels, balms, oils or solids. Single or multiple phase compositions are envisaged. Multiple phase systems include but are not limited to microemulsions, emulsions, and products with discrete separate phases. Emulsions include water-in-oil, oil-in-water emulsions and multiple emulsions (water in oil in water or oil in water in oil for example). Products with discrete separate phases include bi or triphasic systems where the individual water or oil phases can be visibly seen. Preferably the composition is a gel or an emulsion. Preferably the emulsion is an oil-in-water emulsion (where the active agent is a peptide).

Where the composition is aqueous, it preferably comprises from 10% to 99.9% by weight water. In a preferred embodiment, aqueous compositions comprise from 20% to 80 % by weight water. In a preferred embodiment, aqueous compositions comprise from 40% to 70% by weight water. Where the composition is non-aqueous it preferably comprises 0% to up to 10% water, more particularly from 0.1 to 8%, most preferably from 0.5 to 5% water.

Where the composition is an emulsion it comprises an oil and a water phase. The oil phase of an emulsion can be provided by any suitable oily component. Suitable oils for the oil phase may comprise for example: a) hydrocarbon oils, paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetyl dimethicone; e) fatty acid esters and ethers, such as isopropyl palmitate or isopropyl myristate and polypropylene glycol-15 stearyl ether; f) fatty alcohols, such as cetyl alcohol or stearyl alcohol; or g) mixtures thereof, for example, the blend of waxes available commercially under the trade name Cutina (BASF).

The emulsion may comprise 0.1% to 55% by weight of the emulsion of oil phase. In one embodiment, the emulsion may comprise 3% to 25% by weight of the emulsion of oil phase, more preferably from 5% to 20% by weight of the emulsion of oil phase. In an alternative embodiment, the emulsion may comprise 10% to 50% by weight of the emulsion of oil phase, more preferably from 25-50% by weight of the emulsion of oil phase.

Preferably the oil phase of the emulsion comprises oil at a level between 50% and 100% by weight of the oil phase. More preferably the oil phase comprises oil at a level of from 60% to 100%, more preferably from 70% to 100%, and even more preferably from 80% to 100% by weight of the oil phase. Alternatively, the oil phase of the emulsion may comprise a combination of oil, wax or butter. Waxes and butters are hydrocarbons that consist of long aliphatic alkyl chains and may include aromatic groups. They are generally lipophilic and typically solid or malleable at room temperature. Melting points vary depending on the alkyl chain, chain length and associations. Silicone waxes are preferred type of suitable wax based on alkylmethylsiloxane. Oils are typically lipophilic and liquid at room temperature with lower molecular weights than waxes. Where present wax or butter may be present at up to 40% of the oil phase of the emulsion. More preferably the oil phase may contain wax or butter at levels of up to 20% of the oil phase of the emulsion. In an alternative embodiment the oil phase may contain wax or butter at levels of up to 10% of the oil phase of the emulsion.

Preferably the oil phase of the water-in-oil emulsion comprises a silicone oil. Where present, the silicone-containing oil phase preferably comprises an organo polysiloxane oil. The organopolysiloxane oil for use in the composition may be volatile, non-volatile, or a mixture of volatile and non-volatile silicones. The term "nonvolatile" as used in this context refers to those silicones that are liquid or gel under ambient conditions and have a flash point (under one atmosphere of pressure) of greater than 100°C. The term "volatile" as used in this context refers to all other silicone oils. Suitable organopolysiloxanes can be selected from a wide variety of silicones spanning a broad range of volatilities and viscosities. Examples of suitable organopolysiloxane oils include polyalkylsiloxanes, cyclic polyalkylsiloxanes, and polyalkylarylsiloxanes.

Preferred for use herein are organopolysiloxanes selected from the group consisting of: polyalkylsiloxanes, alkyl substituted dimethicones, cyclomethicones, trimethylsiloxysilicates. dimethiconols, polyalkylaryl siloxanes, and mixtures thereof. More preferred for use herein are polyalkylsiloxanes and cyclomethicones. Preferred among the polyalkylsiloxanes are dimethicones.

Alternatively, the silicone oil may be a silicone elastomer. Suitable for use herein are silicone elastomers which can be emulsifying or non-emulsifying crosslinked siloxane elastomers or mixtures thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon- bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester.

Preferably the oil phase comprises silicone, and most preferably, a silicone elastomer. Preferably, the emulsion composition includes from 20% to 35%, by weight of the emulsion composition, of the silicone elastomer raw material.

When the composition is a water-in-oil emulsion it preferably comprises an emulsifier. In a preferred embodiment, the composition comprises from 0.1% to 10% emulsifier, more preferably from 0.25% to 7.5%, still more preferably from 0.5% to 5%, emulsifier by weight of the composition. The emulsifier helps disperse and suspend the aqueous water phase within the oil phase.

### Emulsifiers

The composition of the present invention may comprise an emulsifier. Suitable emulsifiers include all those suitable for the purpose and known by those skilled in the art for use in skin care products. Preferably these emulsifiers have an HLB value of or less than 14, more preferably from 2 to 14, and still more preferably from 4 to 14.

Silicone emulsifiers are preferred. A wide variety of silicone emulsifiers are useful herein. These silicone emulsifiers are typically organically modified organopolysiloxanes, also known to those skilled in the art as silicone surfactants. The skilled person will appreciate that organically modified organopolysiloxanes comprise one or more hydrophilic groups as well as silicone. Useful silicone emulsifiers include dimethicone copolyols. These materials are polydimethyl siloxanes which have been modified to include polyether side chains such as polyethylene oxide chains, polypropylene oxide chains, mixtures of these chains, and chains comprising moieties derived from both ethylene oxide and propylene oxide. Other examples include alkyl-modified dimethicone copolyols, i.e., compounds which comprise C2-C30 pendant side chains. Still other useful dimethicone copolyols include materials having various cationic, anionic, amphoteric and zwitterionic pendant moieties.

Non-limiting examples of dimethicone copolyols and other silicone surfactants useful as emulsifiers herein include polydimethylsiloxane polyether copolymers with pendant polyethylene oxide side chains, polydimethylsiloxane polyether copolymers with pendant polypropylene oxide side chains, polydimethylsiloxane polyether copolymers with pendant mixed polyethylene oxide and polypropylene oxide side chains, polydimethylsiloxane polyether copolymers with pendant mixed poly (ethylene) (propylene) oxide side chains, polydimethylsiloxane polyether copolymers with pendant organobetaine side chains, polydimethylsiloxane polyether copolymers with pendant carboxylate side chains, polydimethylsiloxane polyether copolymers with pendant quaternary ammonium side chains; and also further modifications of the preceding copolymers comprising pendant C2-C30 straight, branched, or cyclic alkyl moieties. A particularly preferred emulsifier is PEG/PPG-18/18 dimethicone.

Suitable, cetyl dimethicone copolyol is commercially available as a mixture with polyglyceryl-4 isostearate (and) hexyl laurate or as a mixture with hexyl laurate and polyglyceryl-3 oleate. Other nonlimiting examples of dimethicone copolyols also include lauryl dimethicone copolyol, dimethicone copolyol acetate, diemethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate.

Among the non-silicone-comprising emulsifiers useful herein are various non-ionic and anionic emulsifying agents such as sugar esters and polyesters, alkoxylated sugar esters and polyesters, Cl-C30 fatty acid esters of Cl-C30 fatty alcohols, alkoxylated derivatives of Cl-C30 fatty acid esters of Cl-C30 fatty alcohols, alkoxylated ethers of Cl-C30 fatty alcohols, polyglyceryl esters of Cl-C30 fatty acids, Cl-C30 esters of polyols, Cl-C30 ethers of polyols, alkyl phosphates, polyoxyalkylene fatty ether phosphates, fatty acid amides, acyl lactylates, soaps, and mixtures thereof. Nonlimiting preferred examples of these non-silicon-comprising emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

### Further peptides

The compositions of the present invention may comprise (further) peptides. Preferably said (additional) peptides are selected from the group consisting of dipeptides, tripeptides, (additional) tetrapeptides, pentapeptides, and mixtures thereof. By tripeptides, it is meant compound comprising an uninterrupted sequence of three amino acids. By tetrapeptides, it is meant a compound comprising an uninterrupted sequence of four amino acids and when using (further tetrapeptides), the tetrapeptides are referred to as '(additional) tetrapeptides'. By pentapeptide it is meant a compound comprising an uninterrupted sequence of five amino acids.

Dipeptides:
The compositions of the present invention may comprise a dipeptide selected from the group consisting of acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide 17, palmitoyl dipeptide 5 diaminobutyroyl hydroxythreonine, palmitoyl dipeptide 5 diaminohydroxybutyrate, palmitoyl dipeptide 7, and mixtures thereof.

Dipeptides are preferably incorporated into the cosmetic composition of the present invention at a level of from 0.1 to 50000ppm, more preferably from 1 to 5000 ppm, most preferably from 10 to 500ppm.

Tripeptides:
The emulsions of the present invention preferably comprise a tripeptide. Said tripeptide may be naturally occurring or of synthetic origin. Suitable tripeptides include tripeptide 1, 2, 3, 4, 5, 6, 7, 8, 9, 10,11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, 41, 42, 43, 44, 45, 46, derivatives thereof and mixtures thereof.

Particularly preferred tripeptides comprise one or more His-based tripeptides. However, another suitable tripeptide may be Arg-Lys-Arg. Particularly preferred tripeptides are based on the structure Gly-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., Gly-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides in accordance with the present invention, (and therefore also the more generic term tripeptides). Derivatives of GHK-tripeptides in accordance with the present invention include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

His-based tripeptides include at least one Histadine amine acid. The other two amino acids in the sequence may be the same or different. Thus, contemplated are, without limitation, His-Xaa-Xaa, His-Xaa-Xbb, His-Xbb-Xaa, Xbb-His-Xbb, Xbb-His-Xaa, Xaa-His-Xbb, Xaa-Xaa-His, Xaa-Xbb-His, Xbb-Xaa-His and Xbb-Xbb-His, where Xaa and Xbb are two different amino acids, although either can be His. Preferably, at least one of the other amino acids is Gly, beta-Ala, Ala, Val, Leu, Pro, Sarcosine (Sar) or Ile. Preferably, at least one of the other amino acids is Pro, Lys, Arg, His, Desmosine and Isodesmosine. Most preferably, Lys is replaced with Orn, Arg, or Citrulline.

Derivatives are also considered to be encompassed by the term His-based tripeptides in accordance with the present invention, (and therefore also the more generic term tripeptides). These derivatives include, inter alia, acyl-derivatives, which are tripeptides substituted with one or more straight-chain or branched-chain, long or short chain, saturated or unsaturated substituted or unsubstituted acyl group(s) having from 1 to 29 carbon atoms. The acyl groups which can be used are the same as those described for the GHK-tripeptides.

Particularly preferred embodiments of tripeptides in accordance with the present invention include N-Acyl-Gly-His-Lys and most preferably, N-Palmitoyl-Gly-His-Lys. Preferred commercially available tripeptide and tripeptide derivative comprising compositions include Biopeptide-CL from SEDERMA, Maxilip(R) from SEDERMA, Biobustyl(R) from SEDERMA.

The tripeptides (or an excipient composition comprising the tripeptide), where included, are preferably incorporated into the cosmetic composition in amounts of from 0.10ppm to 10,000ppm, preferably from 0.50ppm to 5,000ppm, more preferably from 1ppm to 1000ppm, and most preferably from 1ppm to 500ppm. These are again based on a % w/w basis. Thus 100,000ppm is 10% by weight of the emulsion.

Tetrapeptides:
The cosmetic composition may comprise an (additional) tetrapeptide. These may be one or more rigin-based tetrapeptides, one or more ALAMCAT-tetrapeptides or mixtures thereof. These tetrapeptides may be naturally occurring or of synthetic origin. Suitable tetrapeptides for use in the present composition include those selected from the group consisting of well-known tetrapeptide 1, 2, 3, 4, 5, 6, 7, 8, 9, 10, 11, 12,13, 14, 15, 16, 17, 18, 19 ,20, 21, 22, 23, 24, 25, 26, 27, 28, 29, 30, 34, 35, derivatives thereof, and mixtures thereof.

Rigin-based tetrapeptides in accordance with the present invention are based on the structure Gly-Gln-Pro-Arg (Rigin) and include its analogs and derivatives thereof. Rigin is an (additional) tetrapeptide. Analogs of the tetrapeptide rigin useful in accordance with the present invention include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc (SEQ ID No. 57), Xaa-Xbb-Xcc-Pro (SEQ ID No. 58), Xaa-Xbb-Pro-Arg (SEQ ID No. 59), wherein Xaa-Xbb-Pro-Xcc (SEQ ID No. 60), Xaa-Xbb-Xcc-Arg (SEQ ID No. 61), Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-base tetrapeptides, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO3H, SH or S-S.

Derivatives are also considered to include peptide-divalent ion complexes. Cu2+-peptide derivatives are preferred. Cu2+-peptide derivatives may provide an increased biological effect compared to the peptide alone.

ALAMCAT tetrapeptides are tetrapeptides which include at least one amino acid including an aliphatic group comprising side chain. These amino acids include, without limitation, Gly, beta-Ala, Ala, Val, Leu, Sarcosine (Sar) and Ile. These tetrapeptides also include at least one amino acid including at least one NH2 comprising side chain. These amino acids include a side chain that has an amine group that is predominantly uncharged at neutral pH (pH 6-7) such as, without limitation, Gln, Asn, Lys, Orn, 5-hydroxyproline, Citrulline and Canavanine. The ALAMCAT-tetrapeptides also include at least one amino acid having at least one side chain including at least one cationic amine (predominant species is charged such as NH3+, NH2+, etc.-basic amino acids which are positively charged at pH 6.0). These amino acids include, without limitation, Pro, Arg, Lys, His, Desmosine and Isodesmosine. The remaining amino acid can be any amino acid, but is preferably one comprising an alphatic group, pendant amino group or pendant cationic group. Derivatives are also considered to be encompassed by the term ALAMCAT-tetrapeptides in accordance with the present invention, (and therefore also the more generic term tetrapeptides). These derivatives include, inter alia, acyl-derivatives, which are tetrapeptides substituted with one or more straight-chain or branched-chain, substituted or unsubstituted long or short chain, saturated or unsaturated acyl group(s) having from 1 to 29 carbon atoms. The acyl groups which can be used are the same as those described for the rigin-based tetrapeptides.

Preferred embodiments include Peptide E, arg-ser-arg-lys (SEQ ID No. 62), N-acyl-Gly-Gln-Pro-Arg peptides (SEQ ID No. 63), most preferably N-palmitoyl-Gly-Gln-Pro-Arg (SEQ ID No. 64).

Preferred commercially available sources of tetrapeptides include RIGIN, EYELISS, Haloxyl, and MATRIXYL 3000, which comprise between 50 to 500 ppm of palmitoyl-Gly-Gln-Pro-Arg, and other ingredients, such as peptides, chalcones and an excipient, commercially available from SEDERMA, France. Tego Pep 417 available from Evonik. These may be used to produce compositions of the present invention by adding thereto at least one tripeptide as described herein.

The (additional) tetrapeptides when used are preferably incorporated into the cosmetic composition in amounts from 0.1 ppm (0.00001% w/w also referred to herein as "weight percent", "weight %" or simply by weight) to 10,000 ppm (0.5% w/w), preferably from 0.5 ppm to 1000 ppm (0.05% w/w), and most preferably from 1 ppm to 500ppm by weight of the composition.

The combination of tripeptides and (additional) tetrapeptides, can be particularly preferred. When present, the preferred ratio of (additional) tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of (additional) tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of (additional) tetrapeptide used when considered in terms of their amounts in parts per million, again based on overall weight of the composition. In a particularly preferred embodiment, the cosmetic composition of the present invention comprises an (additional) tetrapeptide of the sequence Gly-Gln-Pro-Arg (SEQ ID No. 65), its analogs and derivatives in combination with one or more tripeptide of the sequences Gly-His-Lys, its analogs and derivatives.

### Pentapeptides

The compositions of the present invention may optionally comprise a pentapeptide, derivatives of pentapeptides, and mixtures thereof. As used herein, "pentapeptides" refers to both the naturally occurring pentapeptides and synthesized pentapeptides. Also useful herein are naturally occurring and commercially available compositions that comprise pentapeptides. Suitable pentapeptides are those selected from the group consisting of pentapeptide 1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof, and mixtures thereof.

Suitable pentapeptides for use herein are the pentapeptide, lys-thr-thr-lys-ser (SEQ ID No. 66), Arg-asp-lys-tyr-val (pentapeptide -1; (SEQ ID No. 67)) and derivatives thereof. A preferred commercially available pentapeptide derivative-comprising composition is Matrixyl which comprises 100 ppm of palmitoyl-lys-thr-thr-lys-ser (SEQ ID No. 68) and is commercially available from Sederma, France.

The pentapeptides and/or pentapeptide derivatives where present in the cosmetic composition are preferably included at amounts of from 0.1 ppm (0.00001% w/w t) to 10,000 ppm (0.5% w/w), preferably from 0.5 ppm to 1000 ppm (0.05% w/w), and most preferably from 1 ppm to 500ppm (0.025% w/w) by weight of the composition.

### Matrix metalloproteinase inhibitors (MMPi)

The term "matrix metalloproteinase inhibitor" relates to all molecules and/or plant or bacterial extracts having an inhibitory activity on at least one of the matrix metalloproteinases expressed or synthetized by or in the skin. The family of the matrix metalloproteinases is formed of several well-defined groups on the basis of their resemblance regarding structure and substrate specificity (Woessner J. F. 1991, Faseb Journal, vol. 5,-, 2145). Among these groups, there are collagenases able to degrade fibrillar collagens (MMP-1 or interstitial collagenase, MMP-8 or neutrophil collagenase, MMP- 13 or collagenase 3, MMP-18 or collagenase 4), gelatinases degrading type IV collagen or other denatured collagen form (MMP-2 or A gelatinase (72 kDa), MMP-9 or B gelatinase (92 kDa)), stromelysins (MMP-3 or stromelysin 1, MMP- 10 or stromelysin 2, MMP-11 or stromelysin 3) whose broad spectrum of activity targets proteins of the extracellular matrix such as glycoproteins (fibronectin, laminin), proteoglycanes etc., matrilysin (MMP-7), metalloelastase (MMP- 12) or metalloproteinases (MMP- 14, MMP- 15, MMP- 16 and MMP- 17). Metalloproteinases (MMPs) are proteases that use a metal, (mostly zinc) coordinated to 3 cysteine residues and to a methionine in their active site, that degrade macromolecular components of the extracellular matrix and of basal layers at neutral pH (collagen, elastin, etc ...). This group of enzymes is inactivated by metal chelators. The principal activity regulators of MMPs are the tissue inhibitors of metalloproteinases or TIMPs such TIMP-I, TIMP-2, TIMP-3 and TIMP-4 (Woessner J. F., Faseb Journal, 1991). Furthermore, MMP expression is also regulated by growth factors, cytokines, oncogene products (ras, jun), or also matrix constituents.

The term "matrix metalloproteinase inhibitors" according to the present invention means all molecules able to reduce the MMP's activity regarding the gene expression (transcription and translation) or regarding the activation of the zymogen form of the MMP, or else regarding the local control of active forms. Furthermore, the metalloproteinase inhibitors according to the present invention can also be MMP-1 inhibitors of natural or synthetic origin. The terms "natural origin" or "synthetic origin" mean both a metalloproteinase inhibitor at a pure state or in solution at different concentrations, but natural origin termed inhibitors are obtained by different extraction methods from a natural element (for example lycopene from a tomato) whereas the inhibitors of synthetic origin are all obtained via chemical synthesis

Preferred MMPi are selected from the group consisting of: retinoid, N-acetyl cysteine, glutathione, 2-furildioxime, vitamin C, flavones, isoflavones, hydrolysed rice protein, alfalfa extract, white lupin, zizyphus jujube extract, dihydroxy methyl chromone, kudzu extract, vitis vinifera extract, Oenothera biennis extract Anogeissus leiocarpus extract, and mixtures thereof.

Where present, MMPi are present at a level of from 0.01% to 10%, more preferably 0.1% to 5% and most preferably from 0.5% to 2.5% by weight of the cosmetic composition.

### Skin Conditioning Agent

The compositions of the present invention may optionally comprise a skin conditioning agent. Said skin conditioning agents may preferably be selected from the group consisting of: humectants, emollients, moisturisers, and mixtures thereof. Where present, they are preferably present at a level of from 0.01% to 20%, more preferably from 0.1% to 10%, most preferably from 0.5% to 7% by weight of the cosmetic composition.

Preferred skin conditioning agents are selected from the group consisting of: guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, hyaluronic acid and salts thereof, lactamide monoethanolamine, acetamide monoethanolamine, panthenol, allantoin, and mixtures thereof.

More preferably said skin conditioning agent is selected from the group consisting of: glycerine, arabinogalactan, butylene glycol, hyaluronic acid, shea butter, propylene glycol, ethylhexyl glycerine, hyaluronate and mixtures thereof.

### Antioxidant Agent

The compositions of the present invention may optionally comprise an antioxidant agent. Suitable antioxidant agents may include: a) ascorbic acid its salts, esters, glucosides and glucosamines, particularly sodium ascorbyl phosphate, magnesium ascorbyl phosphate and ascorbyl palmitate b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts, particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, panax ginseng, such as that available under the trade name "Panax ginseng 1.1 extract 4294"from S Black Ltd or "Phytexcell Panax ginseng" available from Croda Chemicals Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica ^{™} by Merck Speciality chemicals, and Grape Seed oil, such as that available from Chesham Chemicals Limited.

The amounts of antioxidant agents used in the cosmetic composition are expressed as dry weights, as understood by a man skilled in the art. The total amount of antioxidant agents optionally present in the composition may range from 0.005% to 10% by weight, preferably 0.5% to 5%, most preferably 0.2% to 1.5% by weight of the composition.

Particularly preferred synergistic combinations of antioxidant agents suitable for inclusion in the cosmetic composition of the present invention are two or more members selected from the group consisting of : i) panax ginseng, morus alba and magnesium ascorbyl phosphate; ii) panax ginseng, morus alba and sodium ascorbyl phosphate; iii) panax ginseng, morus alba and rosmarinus officinalis; iv) ginkgo biloba, phyllanthus emblica and Dimethylmethoxy chromanol; v) morus alba, camellia sinensis and dimethylmethoxy chromanol; vi) morus alba, camellia sinensis and tocopheryl acetate; vii) panax ginseng, morus alba and origanum vulgare.

In these preferred combinations (a) the panax ginseng is preferably present in an amount of 0.005% to 0.1%, more preferably 0.01% to 0.05% by weight of the composition; (b) the morus alba is preferably present in an amount of 0.0005% to 0.01%, more preferably 0.001% to 0.005% by weight of the composition; (c) the sodium,magnesium ascorbyl phosphate or ethyl ascorbic acid is preferably present in an amount of 0.05% to 2.5%, preferably 0.1% to 2%, most preferably 0.15% to 1.5% by weight of the composition;(d) the rosmarinus officinalis or origanum vulgare or phyllanthus emblicais preferably present in an amount of 0.01% to 0.5%, more preferably 0.05% to 0.2% by weight of the composition e) the dimethylmethoxy chromanol is preferably present in an amount of 0.0005% to 0.1%, more preferably from 0.005% to 0.05% by weight of the composition ; f) the camellia sinensis is preferably present in an amount of 0.005% to 0.2%, more preferably from 0.01% to 0.1% and the g) Tocoperol acetate is preferably present in an amount of 0.01 to 0.5%, more preferably from 0.05% to 0.25%.

### Vitamins

The compositions of the present invention may comprise one or more vitamins. The compositions may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid. The composition may comprise vitamin B, vitamin B derivatives, vitamin B1 to vitamin B12 and their derivatives. In a further embodiment the composition comprising the Vitamin B3 derivative niacinamide.

In an alternative embodiment, the cosmetic composition comprises vitamin K, vitamin K derivatives, vitamin H, vitamin D, vitamin D derivatives and mixtures thereof. In an alternative embodiment, the cosmetic composition comprises vitamin E, vitamin E derivatives such as tocopherol and tocopheryl acetate, and provitamins thereof, such as panthenol and mixtures thereof.

In a further embodiment, the present cosmetic composition comprises retinoid compounds, including retinoic acid, retinaldehyde, retinol and derivatives thereof. In another embodiment, the cosmetic composition comprises retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e. g. plant) sources. In one embodiment, when vitamin compounds are present in the compositions of the instant invention, the emulsion compositions comprise from about 0.0001% to 50%, more preferably from 0.001% to 10%, still more preferably from 0.01% to 8%, and still more preferably from 0.1% to 5%, by weight of the composition, of the vitamin compound.

### Salicylic Acid Compound

The compositions of the present invention may comprise a salicylic acid compound, its esters, its salts, or combinations thereof. In one embodiment of the compositions of the present invention, the salicylic acid compound preferably comprises from 0.0001% to 25%, more preferably from 0.001% to 15%, even more preferably from 0.01% to 10%, still more preferably from 0.1% to 5%, and even more preferably from 0.0 1% to 2%, more preferably 0.1% to 2% by weight of the composition, of salicylic acid.

### Sunscreen

The compositions of the present invention may optionally comprise a sunscreen component. The sunscreen may comprise organic or inorganic sun filters or a combination of the two. Suitable inorganic sun filters include those selected from the group consisting of: microfine titanium dioxide, microfine zinc oxide, boron nitride and, mixtures thereof.

Suitable organic sunscreens include those selected from the group consisting of : a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4- (tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino- (p-caibo-2-ethyl-hexyl-1-oxi)-1, 3,5 triazine, h) camphor derivatives such as methylbenzylidene camphor and i) mixtures thereof. Other preferred sunscreen ingredients include those selected from the group consisting of: homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreen agent is optionally present in an amount from 0.1 to 10% by weight of the composition.

### Other Optional Ingredients

The compositions of the present invention may also optionally comprise one or more of the following optional ingredients. Preservatives may be added to the emulsion such as 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, isothiazolone, sodium benzoate, chlorhexidine digluconate and sodium propyl paraben, suitably in an amount of from 0.01% to 10% by weight of the emulsion.

Thickeners, viscosity modifying agents and/or gelling agents may be added to the emulsion composition, such as acrylic acid polymers e. g. available commercially under the trade name Carbopol, Novethix, EZ 4U or Ultrez (Lubrizol) or Sepigel, Sepiplus and Simulgel (Seppic). Also modified cellloses e. g. hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name"Pluronic"RTM), PVM, MA, or a decadiene crosspolymer (available under the trade name Stabilez 60), ethoxylated fatty alcohols, salt (magnesium chloride, sodium chloride), Aristoflex AVC (Clariant), phthalic acid amide, xanthan gum, sodium polyacrylate, polyvinyl alcohols, fatty alcohols and alkyl galactomannans available under the trade name N-Hance from Hercules, suitably in an amount of from 0.1% to 10% by weight of the composition.

Sequestering agents may be added to the emulsion composition, such as ethylenediamine tetraacetic acid and salts thereof, suitably in an amount of from 0.005% to 0.5% by weight of the composition.

The composition may also include waxes such as cocoa butter suitably in an amount of from 1% to 99% by weight of the composition.

The composition may also comprise suitable, cosmetically acceptable diluents, carriers and/or propellants such as dimethyl ether.

The composition may also include pearlising agents such as stearic monoethanolamide and/or mica, suitably in an amount of from 0.01% to 10% by weight of the composition.

Perfumes may be added suitably in an amount of from 0.01% to 2% by weight of the composition, as may water soluble dyes such as tartrazine, suitably in an amount of from a trace amount (such as 1 x 10-5 %) to 0.1 % by weight of the composition.

The composition may also include pH adjusting agents such as sodium hydroxide, aminomethyl propanol, triethanolamine, suitably in an amount of from 0.01 % to 10% by weight of the composition. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH between 3 and 10, preferably between 4 and 8.

### Method of use

The method may comprise administering or delivering a therapeutically effective amount of the composition comprising the biologically active agent (e.g., a peptide). Administering or delivering may comprise contacting the composition with skin of the subject. Preferably, the skin is facial skin of the subject.

The subject may be a mammal. Preferably, the subject is a human being.

### Use

According to another aspect of the invention, there is provided a use of one or more solvents of the invention to dissolve a biologically active agent (e.g., a peptide). The one or more solvents may be selected from the solvents disclosed herein. Preferably, the one or more solvents is selected from the group consisting of a dimethyl silane; a monosaccharide ether; and mixtures thereof.

The term *"comprising"* may refer to *"consisting of*" or *"consisting essentially of*" .

### Examples

The FFE tool was used to identify ingredients which could help deliver the biologically active peptides deeper into skin and therefore increase their efficacy.

FFE was used by first entering the HSP (Hansen Solubility Parameters), molecular volume and Melting point values or simplified molecular-input line-entry system (SMILES) of the peptide into the software (if not already preloaded).

Then, in the formulation part of the FFE software, various options were chosen to model the behaviour of the peptide with a range of preloaded ingredients or ingredients that we added via SMILES/HSP values.

As the peptides were predominately water soluble, optimisation focused on the water phase of the formulation. In short, optimisation involved looking at the materials on the database and making a judgement on the materials/levels that are appropriate for the formulation and comparing improvement of skin penetration to known and used formulations.

### Results

### Example 1 - Cosmetic materials to improve the Penetration of Pal-GHK

| **System** | **Active On** | **Active In** | **Active Out** |
|---|---|---|---|
| Glycerin 81.51% | 0.00417 | 0.00003 | 0.00000 |
| Butylene Glycol 18.49% | | | |
| Baseline humectant water phase | | | |
| Glycerin 48.50% | 0.00408 | 0.00012 | 0.00000 |
| **PEG-7 Glyceryl Cocoate 33.01%** | | | |
| Butylene Glycol 18.49 | | | |
| Glycerin 48.50% | 0.00437 | 0.0005 | 0.00000 |
| Butylene Glycol 18.49% | | | |
| **Methyl Gluceth-20 33.01%** | | | |
| Glycerin 48.50% | 0.00389 | 0.00031 | 0.00000 |
| Butylene Glycol 18.49% | | | |
| **Bis-PEG-18 Methyl Ether Dimethyl** | | | |
| **Silane 33.01%** | | | |

The PEG-7 Glyceryl Cocoate, Methyl Glucerth-20 and bis-PEG-18 methyl ether dimethyl silane all increased theorical the amount of Pal GHK into the skin. Used at cosmetically acceptable levels without negativity effecting textural attributes.

### Example 2 - Understand effect of Methyl Gluceth-20 with Pal-LSVD and Pal-GPKG in a cosmetic water phase humectant system

| **System** | **Peptide** | **Active On** | **Active In** | **Active Out** |
|---|---|---|---|---|
| Glycerin 43.55% | Pal-LSVD | 0.00496 | 0.00004 | 0.00000 |
| Butylene Glycol 56.45% | (SEQ ID No. 51) | | | |
| Baseline humectant water phase | | | | |
| Glycerin 29.75% | Pal-LSVD | 0.00493 | 0.00007 | 0.00000 |
| Butylene Glycol 40.66% | (SEQ ID No. 51) | | | |
| **Methyl Gluceth-20 29.75%** | | | | |
| Glycerin 43.55% | Pal-GPKG (SEQ ID No. 69) | 0.00495 | 0.00005 | 0.00000 |
| Butylene Glycol 56.45% | | | | |
| Baseline humectant water phase | | | | |
| Glycerin 29.75% | Pal-GPKG | 0.00493 | 0.00007 | 0.00000 |
| Butylene Glycol 40.66% | (SEQ ID No. 69) | | | |
| **Methyl Gluceth-20 29.75%** | | | | |

## Claims

1. A composition comprising a biologically active agent and one or more solvents, which dissolve the biologically active agent, selected from the group consisting of a dimethyl silane, a monosaccharide ether, and a mixture thereof.

2. The composition of claim 1, wherein the solvent is a dimethyl silane.

3. The composition of claim 1, wherein the solvent is a monosaccharide ether.

4. The composition of claim 1 or claim 2, wherein the dimethyl silane is or comprises an ether of dimethyl silane (ether dimethyl silane).

5. The composition of claim 4, wherein the ether of dimethyl silane is bis-PEG-18 methyl ether dimethyl silane.

6. The composition of claim 1, wherein the monosaccharide ether is or comprises an alkoxylated alkyl monosaccharide ether.

7. The composition of claim 6, wherein the alkoxylated alkyl monosaccharide ether is or comprises an ethoxylated methyl monosaccharide ether.

8. The composition of claim 7, wherein the ethoxylated methyl monosaccharide ether is or comprises methyl-gluceth-20.

9. The composition according to any one of the preceding claims, wherein the agent is or comprises a peptide.

10. The composition according to claim 9, wherein the agent is or comprises a matrikine.

11. The composition of claim 10, wherein the peptide is a lipo-matrikine

12. The composition of claim 11, wherein the lipo-matrikine is a palmityl matrikine.

13. A method of delivering a composition to the skin of a subject, the method comprising contacting a composition according to any one of the preceding claims with the skin of said subject.

14. Use of a solvent selected from the group consisting of a dimethyl silane, a monosaccharide ether, and mixtures thereof, to dissolve a biologically active agent.
